# FASCICULE DE BREVET EUROPEEN

(11) **EP 4 337 392 B1**
(45) Date de publication et mention de la délivrance du brevet: **14.05.2025**
(21) Numéro de dépôt: 22711578.9
(22) Date de dépôt: 28.02.2022
(51) Int. Cl.: B05B 12/08, A61M 15/08, G01F 1/68

(54) **PROCÉDÉ ET DISPOSITIF D'ANALYSE D'UN DISPOSITIF DE PULVÉRISATION DE PRODUIT FLUIDE PHARMACEUTIQUE**
VERFAHREN UND VORRICHTUNG ZUR ANALYSE EINER VORRICHTUNG ZUM SPRÜHEN EINES PHARMAZEUTISCHEN FLÜSSIGPRODUKTS
METHOD AND DEVICE FOR ANALYSING A DEVICE FOR SPRAYING A PHARMACEUTICAL FLUID PRODUCT

(30) Priorité: 10.05.2021 FR 2104936
(43) Date de publication de la demande: 20.03.2024
(73) Titulaire: APTAR France SAS, 27110 Le Neubourg (FR)
(72) Inventeur: WYART, Rémy, 27130 BALINES (FR)
(74) Mandataire: CAPRI
(86) Numéro de dépôt international: PCT/FR2022/050354
(87) Numéro de publication internationale: WO 2022/238628

(56) Documents cités:
- DE-A1- 102017 218 677
- JP-A- H02 130 260
- JP-A- S54 127 347
- KR-A- 20040 044 649
- KR-U- 19980 056 607
- US-A- 5 753 806
- US-A1- 2016 216 108

## Description

La présente invention concerne un dispositif et un procédé d'analyse de spray généré par un dispositif de pulvérisation de produit fluide pharmaceutique.

Les dispositifs de pulvérisation de produit fluide pharmaceutique sont bien connus. Ils comportent généralement une tête de pulvérisation pourvue d'un orifice de pulvérisation, assemblée sur un réservoir contenant le produit fluide à distribuer. En particulier dans des applications de pulvérisation nasale, l'efficacité thérapeutique du produit fluide pulvérisé peut dépendre des propriétés du spray généré lors de l'actionnement du dispositif. De manière connue, à la fin de la chaîne de montage, c'est-à-dire lorsque le dispositif de pulvérisation est assemblé, et juste avant d'être expédié chez le fabriquant du produit fluide pharmaceutique pour y être assemblé sur un réservoir correspondant, un certain nombre d'échantillons de dispositifs assemblés sont testés en laboratoire pour vérifier si les propriétés du spray correspondent au cahier des charges prédéfini.

Un inconvénient de ce système est qu'il concerne des dispositifs assemblés, et donc destructeur de ces dispositifs qui ne pourront plus, après avoir été testés, être livrés au client.

De plus, ce système impose une vérification humaine des dispositifs testés, et n'est donc pas totalement automatisable.

Pour surmonter cet inconvénient, le document WO2018130791 propose la visualisation par strioscopie d'un flux d'air comprimé chaud ou froid envoyé à travers une tête de pulvérisation. Cette méthode permet de distinguer un spray d'un jet, mais ne permet pas d'évaluer la géométrie et/ou la symétrie du spray, et donc sa conformité par rapport à des spécifications données. Cette méthode a de plus l'inconvénient d'avoir à prévoir un banc strioscopique, relativement complexe et coûteux.

Les documents US2016216108, JPS54127347 et US5753806 décrivent d'autres dispositifs de l'état de la technique.

La présente invention a pour but de surmonter les inconvénients susmentionnés.

En particulier, la présente invention a pour but de fournir un dispositif et un procédé d'analyse d'un dispositif de pulvérisation de produit fluide pharmaceutique qui permet de détecter les dispositifs non conformes par rapport à des spécifications prédéterminées.

La présente invention a aussi pour but de fournir un dispositif et un procédé d'analyse qui soit non destructeur des dispositifs testés.

La présente invention a également pour but de fournir un dispositif et un procédé d'analyse qui soit automatisé en grande partie.

La présente invention a aussi pour but de fournir un dispositif et un procédé d'analyse qui permette de tester un grand nombre, notamment 100%, des dispositifs de pulvérisation, sans ralentir la chaine de montage de manière substantielle.

La présente invention a également pour but de fournir un dispositif et un procédé d'analyse qui soit simple et/ou peu coûteux à fabriquer, à assembler et à utiliser.

La présente invention a donc pour objet un procédé d'analyse d'un dispositif de pulvérisation de produit fluide pharmaceutique, comprenant les étapes suivantes:
- fournir une tête de pulvérisation d'un dispositif de pulvérisation de produit fluide pharmaceutique, ladite tête de pulvérisation comportant un orifice de pulvérisation,
- fournir une surface de réception comportant une pluralité de capteurs,
- faire passer un flux de gaz comprimé à travers ledit orifice de pulvérisation de ladite tête de pulvérisation,
- envoyer ledit flux de gaz comprimé sur ladite surface de réception,
- visualiser la zone d'impact dudit flux de gaz comprimé sur ladite surface de réception au moyen desdits capteurs, et
- analyser ladite visualisation de ladite zone d'impact pour déterminer si ladite zone d'impact est conforme ou non conforme à des spécifications prédéterminées,

ladite surface de réception étant de forme sphérique au moins au niveau de la zone d'impact dudit flux de gaz comprimé,
ladite étape d'analyser comprenant de déterminer la géométrie, notamment la symétrie, de la zone d'impact dudit flux de gaz comprimé sur ladite surface de réception.

Avantageusement, ledit flux de gaz comprimé est un flux d'air comprimé.

Avantageusement, lesdites spécifications prédéterminées comprennent une étendue planaire prédéterminée de la zone d'impact dudit flux de gaz comprimé sur ladite surface de réception, de sorte que les têtes de pulvérisation pour lesquelles ladite étendue planaire est similaire à ladite étendue planaire prédéterminée sont classées conformes, et les têtes de pulvérisation pour lesquelles ladite étendue planaire est différente de ladite étendue planaire prédéterminée sont classés non conformes.

Avantageusement, ladite surface de réception est formée par la surface interne d'une partie de sphère, notamment d'une demi-sphère.

Avantageusement, ladite surface de réception comporte une pluralité d'ouvertures, chacune étant reliée à un capteur respectif.

Avantageusement, lesdits capteurs forment un réseau en nid d'abeille comportant au moins deux zones de capteurs concentriques.

La présente invention a également pour objet un dispositif d'analyse d'un dispositif de pulvérisation de produit fluide pharmaceutique, comprenant:
- une tête de pulvérisation d'un dispositif de pulvérisation de produit fluide pharmaceutique, ladite tête de pulvérisation comportant un orifice de pulvérisation,
- une surface de réception comportant une pluralité de capteurs,
- des moyens de génération d'un flux de gaz comprimé pour faire passer un flux de gaz comprimé à travers ledit orifice de pulvérisation de ladite tête de pulvérisation sur ladite surface de réception,
- des moyens de visualisation pour visualiser au moyen desdits capteurs la zone d'impact dudit flux de gaz comprimé sur ladite surface de réception, et
- des moyens d'analyse pour analyser ladite visualisation de ladite zone d'impact pour déterminer si ladite zone d'impact est conforme ou non conforme à des spécifications prédéterminées,

ladite surface de réception étant de forme sphérique au moins au niveau de la zone d'impact dudit flux de gaz comprimé,
lesdits capteurs formant un réseau en nid d'abeille comportant au moins deux zones de capteurs concentriques.

Avantageusement, ledit flux de gaz comprimé est un flux d'air comprimé.

Avantageusement, ladite surface de réception est formée par la surface interne d'une partie de sphère, notamment d'une demi-sphère.

Avantageusement, ladite surface de réception comporte une pluralité d'ouvertures, chacune étant reliée à un capteur respectif.

Avantageusement, un filtre est disposé devant chaque capteur.

Avantageusement, ledit réseau de capteurs comprend une zone centrale avec un seul capteur central, et une première zone concentrique, disposée radialement à l'extérieur de ladite zone centrale, avec six capteurs.

Avantageusement, ledit réseau de capteurs comprend une seconde zone concentrique, disposée radialement à l'extérieur de ladite première zone concentrique, avec douze capteurs.

Avantageusement, ledit réseau de capteurs comprend une troisième zone concentrique, disposée radialement à l'extérieur de ladite seconde zone concentrique, avec dix-huit capteurs.

Avantageusement, lesdits capteurs sont proches les uns des autres, formant ainsi un réseau régulier et dense de capteurs sur ladite surface de réception.

Avantageusement, lesdits moyens de génération du flux de gaz comprimé sont adaptés à générer des impulsions de durée réglable, notamment de 50 à 300 ms.

Avantageusement, lesdits capteurs sont des capteurs de débit massique.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un dispositif pour analyser un dispositif de pulvérisation, selon un mode de réalisation avantageux, et
la figure 2 est une représentation schématique d'une zone d'impact conforme.

Un objectif de l'invention est d'améliorer la qualité du contrôle des dispositifs de pulvérisation.

De manière classique, chaque dispositif de pulvérisation comprend une tête de pulvérisation 1 pourvue d'un orifice de pulvérisation 2. Eventuellement, un profil de pulvérisation (non représenté) peut être prévu en amont dudit orifice de pulvérisation 2 pour générer un spray en sortie de l'orifice.

La présente invention prévoit de faire passer un flux de gaz comprimé F à travers une tête de pulvérisation 1, et de diriger ce flux F, sortant de l'orifice de pulvérisation 2 avec la forme d'un spray conique S, vers une surface de réception 10 comportant une pluralité de capteurs 20. Avantageusement, le flux de gaz comprimé F est un flux d'air comprimé, mais il est entendu que selon l'invention, n'importe quel gaz approprié autre que de l'air pourrait être utilisé.

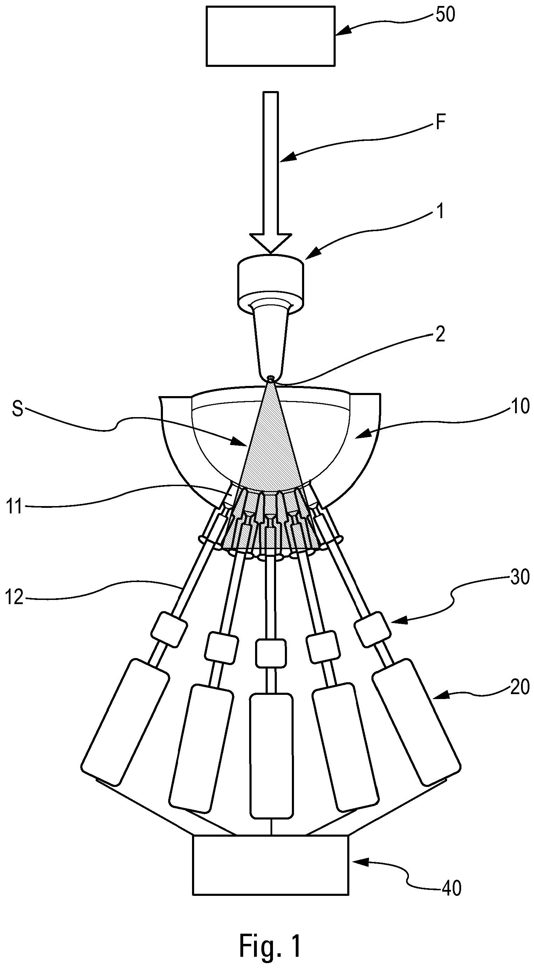

La figure 1 montre un dispositif de test selon un mode de réalisation avantageux.

La tête de pulvérisation 1 est disposée en face d'une surface de réception 10 de forme au moins partiellement sphérique. En particulier, la surface de réception 10 peut être formée par la surface interne d'une partie de sphère, par exemple d'une demi-sphère, comme visible sur la figure 1.

Dans l'exemple de la figure 1, la surface de réception 10 comporte une pluralité d'ouvertures 11, chacune étant reliée à un capteur 20 respectif, par exemple via un tuyau respectif 12. En variante, les capteurs 20 pourraient être sur la surface de réception 10. Du fait de la forme hémisphérique de la surface de réception 10, chaque ouverture 11 est disposée à la même distance de l'orifice de pulvérisation 2.

Eventuellement, un filtre 30 peut être disposé devant chaque capteur 20.

Des moyens de génération 50 d'un flux de gaz comprimé F sont prévus pour faire passer un flux de gaz comprimé F à travers la tête de pulvérisation 1.

Avantageusement, les capteurs 20 et/ou les ouvertures 11 forment un réseau en nid d'abeille comportant au moins deux zones concentriques. Ainsi, le réseau de capteurs 20 peut comprendre une zone centrale avec un seul capteur central, et une première zone concentrique, disposée radialement à l'extérieur de ladite zone centrale, avec six capteurs.

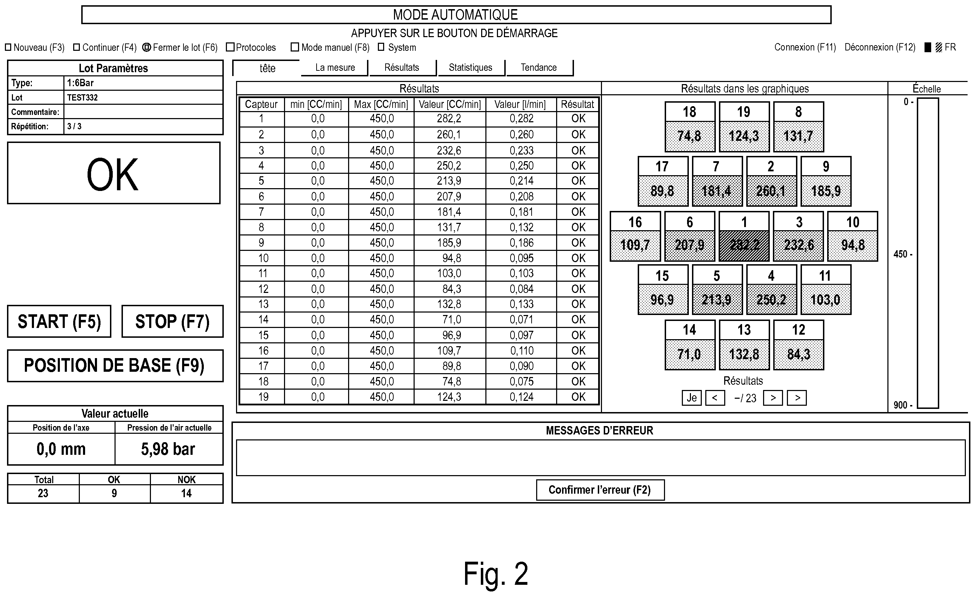

Avantageusement, le réseau de capteurs 20 comprend une seconde zone concentrique, disposée radialement à l'extérieur de ladite première zone concentrique, avec douze capteurs. C'est cette configuration avec dix-neuf capteurs qui est utilisée pour l'analyse représentée sur la figure 2.

Eventuellement, le réseau de capteurs 20 peut comprendre une troisième zone concentrique, disposée radialement à l'extérieur de ladite seconde zone concentrique, avec dix-huit capteurs.

D'autres formes de réseau, par exemple en carré ou en cercle, sont aussi envisageables
La forme particulière de la surface de réception 10, avec une pluralité de capteurs 30 proches les uns des autres, formant ainsi un réseau régulier et dense de capteurs sur ladite surface de réception, permet un contact local du flux de gaz comprimé F sur les capteurs 20, sans dispersions et sans perturbations du flux, ce qui rend visible la zone d'impact avec une grande fiabilité.

Avantageusement, les capteurs 20 sont des capteurs de débit massique. Le choix d'un contrôle de débit massique, s'appuyant sur le principe de conservation de la masse, est le plus judicieux. En effet, le gaz, et notamment l'air, étant un fluide compressible, il ne serait pas pertinent de contrôler un débit volumique. Un capteur de débit massique utilise les calories du fluide (gaz ou liquide) comme conductivité pour déterminer le débit massique. Le capteur est composé d'un tube dans lequel se trouvent deux sondes de température en acier inoxydable et un élément chauffant. La première sonde mesure la température du gaz avant l'élément chauffant et l'autre la température du gaz après l'élément chauffant. Une différence de température ΔT est ainsi créée entre les deux sondes. En l'absence de débit, ce ΔT ne change pas. Lorsqu'un débit existe, l'élément chauffant restitue une quantité de chaleur au gaz en mouvement. Cette perte de chaleur, identifiée grâce aux sondes, est compensée en fournissant davantage d'énergie à l'élément chauffant afin de maintenir un ΔT constant entre les deux sondes. L'énergie requise pour maintenir ce ΔT est proportionnelle au débit massique. En mesurant l'énergie consommée par la sonde il est alors possible de déterminer le débit massique traversé par le capteur de débit.

Néanmoins, d'autres types de capteurs pourraient être utilisés, par exemple des capteurs de pression.

Pour réaliser les évaluations de conformité, on prévoit avantageusement des moyens d'analyse 40 pour analyser les mesures de chaque capteur 30 et ainsi déterminer si la zone d'impact du flux de gaz comprimé F issu de ladite tête de pulvérisation 1 sur la surface de réception 10 est conforme ou non conforme à des spécifications prédéterminées.

La durée de l'impulsion de gaz comprimé F est avantageusement réglable, notamment de 50 à 300 ms.

Avantageusement, on réalise plusieurs cycles successifs sur une même tête de pulvérisation, par exemple cinq cycles. La constance ou répétabilité des résultats permet également d'évaluer la conformité de ladite tête de pulvérisation.

Les spécifications prédéterminées peuvent comprendre une étendue planaire prédéterminée de ladite zone d'impact sur ladite surface de support 10, de sorte que les têtes de pulvérisation 1 pour lesquelles ladite étendue planaire est similaire à ladite étendue planaire prédéterminée sont classées conformes, et les têtes de pulvérisation 1 pour lesquelles ladite étendue planaire est différente de ladite étendue planaire prédéterminée sont classés non conformes. La géométrie, et notamment la symétrie, de la zone d'impact peut aussi être utilisée dans l'évaluation de conformité. D'autres paramètres peuvent aussi être envisagés, tels que les écarts entre les mesures de capteurs adjacents.

Les moyens d'analyse peuvent comprendre des moyens de mesure de la géométrie de la zone d'impact du flux de gaz comprimé F sur la zone de réception 10. Par exemple, on détermine le barycentre de la zone d'impact, et on mesure les distances maximale et minimale de ce barycentre du bord de la zone d'impact. La comparaison de ces distances avec des valeurs prédéterminées permet alors d'évaluer la conformité du dispositif testé. Ainsi, l'évaluation de conformité tient compte non seulement de la surface de la zone d'impact, mais aussi de sa géométrie, en particulier sa symétrie. Ceci permet d'établir qu'un spray sortant d'une tête de pulvérisation conforme aura une forme conique acceptable, tant d'un point de vue de l'angle du spray que de sa symétrie.

La figure 2 illustre une représentation schématique obtenue avec le procédé et le dispositif de l'invention, à partir de laquelle il est possible d'évaluer notamment l'étendue planaire et la géométrie, notamment la symétrie, de la zone d'impact. Ainsi, dans cet exemple qui montre un résultat conforme, on constate que les mesures des différents capteurs sont homogènes dans la répartition spatiale, et avec des écarts entre les différentes zones concentriques du réseau qui sont acceptables, ce qui démontre une bonne répartition géométrique.

La présente invention présente de nombreux avantages, et notamment:
- elle permet un contrôle automatisé de conformité sur divers types de dispositif de pulvérisation;
- elle permet une analyse non destructive desdits dispositifs de pulvérisation;
- elle permet d'analyser 100% des dispositifs de pulvérisation assemblés sur une chaine de montage, sans ralentissement substantiel de celle-ci;
- elle permet de réaliser plusieurs tests successifs sur un même dispositif pour évaluer la répétabilité des résultats;
- elle utilise un montage compacte et facilement adaptable;
- elle utilise des composants simples et standards, donc généralement peu coûteux;
- elle permet un traitement d'image robuste, qui peut être réalisé en temps réel;
- elle assure une bonne répétabilité et une bonne discrimination des dispositifs conformes et non-conformes.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Procédé d'analyse d'un dispositif de pulvérisation de produit fluide pharmaceutique, comprenant les étapes suivantes:
- fournir une tête de pulvérisation (1) d'un dispositif de pulvérisation de produit fluide pharmaceutique, ladite tête de pulvérisation (1) comportant un seul orifice de pulvérisation (2),
- fournir une surface de réception (10) comportant une pluralité de capteurs (20),
- faire passer un flux de gaz comprimé (F) à travers ledit orifice de pulvérisation (2) de ladite tête de pulvérisation (1),
- envoyer ledit flux de gaz comprimé (F) sur ladite surface de réception (10),
- visualiser la zone d'impact dudit flux de gaz comprimé (F) sur ladite surface de réception (10) au moyen desdits capteurs (20), et
- analyser ladite visualisation de ladite zone d'impact pour déterminer si ladite zone d'impact est conforme ou non conforme à des spécifications prédéterminées,
**caractérisé en ce que** ladite surface de réception (10) est de forme sphérique au moins au niveau de la zone d'impact dudit flux de gaz comprimé (F), ladite étape d'analyser comprenant de déterminer la géométrie, notamment la symétrie, de la zone d'impact dudit flux de gaz comprimé (F) sur ladite surface de réception (10).

2. Procédé selon la revendication 1, dans lequel ledit flux de gaz comprimé (F) est un flux d'air comprimé.

3. Procédé selon l'une quelconque des revendications précédentes, dans lequel lesdites spécifications prédéterminées comprennent une étendue planaire prédéterminée de la zone d'impact dudit flux de gaz comprimé (F) sur ladite surface de réception (10), de sorte que les têtes de pulvérisation (1) pour lesquelles ladite étendue planaire est similaire à ladite étendue planaire prédéterminée sont classées conformes, et les têtes de pulvérisation (1) pour lesquelles ladite étendue planaire est différente de ladite étendue planaire prédéterminée sont classés non conformes.

4. Procédé selon l'une quelconque des revendications précédentes, dans lequel ladite surface de réception (10) est formée par la surface interne d'une partie de sphère, notamment d'une demi-sphère.

5. Procédé selon l'une quelconque des revendications précédentes, dans lequel ladite surface de réception (10) comporte une pluralité d'ouvertures (11), chacune étant reliée à un capteur (20) respectif.

6. Procédé selon l'une quelconque des revendications précédentes, dans lequel lesdits capteurs (20) forment un réseau en nid d'abeille comportant au moins deux zones de capteurs concentriques.

7. Dispositif d'analyse d'un dispositif de pulvérisation de produit fluide pharmaceutique, comprenant:
- une tête de pulvérisation (1) d'un dispositif de pulvérisation de produit fluide pharmaceutique, ladite tête de pulvérisation (1) comportant un seul orifice de pulvérisation (2),
- une surface de réception (10) comportant une pluralité de capteurs (20),
- des moyens de génération (50) d'un flux de gaz comprimé (F) pour faire passer un flux de gaz comprimé (F) à travers ledit orifice de pulvérisation (2) de ladite tête de pulvérisation (1) sur ladite surface de réception (10),
- des moyens de visualisation pour visualiser au moyen desdits capteurs (20) la zone d'impact dudit flux de gaz comprimé (F) sur ladite surface de réception (10), et
- des moyens d'analyse (40) pour analyser ladite visualisation de ladite zone d'impact pour déterminer si ladite zone d'impact est conforme ou non conforme à des spécifications prédéterminées,
**caractérisé en ce que** ladite surface de réception (10) est de forme sphérique au moins au niveau de la zone d'impact dudit flux de gaz comprimé (F), lesdits capteurs (20) formant un réseau en nid d'abeille comportant au moins deux zones de capteurs concentriques.

8. Dispositif selon la revendication 7, dans lequel ledit flux de gaz comprimé (F) est un flux d'air comprimé.

9. Dispositif selon la revendication 7 ou 8, dans lequel ladite surface de réception (10) est formée par la surface interne d'une partie de sphère, notamment d'une demi-sphère.

10. Dispositif selon l'une quelconque des revendications 7 à 9, dans lequel ladite surface de réception (10) comporte une pluralité d'ouvertures (11), chacune étant reliée à un capteur (20) respectif.

11. Dispositif selon l'une quelconque des revendications 7 à 10, dans lequel un filtre (30) est disposé devant chaque capteur (20).

12. Dispositif selon l'une quelconque des revendications 7 à 11, dans lequel ledit réseau de capteurs (20) comprend une zone centrale avec un seul capteur central, et une première zone concentrique, disposée radialement à l'extérieur de ladite zone centrale, avec six capteurs.

13. Dispositif selon la revendication 12, dans lequel ledit réseau de capteurs (20) comprend une seconde zone concentrique, disposée radialement à l'extérieur de ladite première zone concentrique, avec douze capteurs.

14. Dispositif selon la revendication 13, dans lequel ledit réseau de capteurs (20) comprend une troisième zone concentrique, disposée radialement à l'extérieur de ladite seconde zone concentrique, avec dix-huit capteurs.

15. Dispositif selon l'une quelconque des revendications 7 à 14, dans lequel lesdits capteurs (20) sont proches les uns des autres, formant ainsi un réseau régulier et dense de capteurs (20) sur ladite surface de réception (10).

16. Dispositif selon l'une quelconque des revendications 7 à 15, dans lequel lesdits moyens de génération (50) du flux de gaz comprimé (F) sont adaptés à générer des impulsions de durée réglable, notamment de 50 à 300 ms.

17. Dispositif selon l'une quelconque des revendications 7 à 16, dans lequel lesdits capteurs (20) sont des capteurs de débit massique.

## Patentansprüche

1. Verfahren zur Analyse einer Vorrichtung zum Sprühen eines fluiden pharmazeutischen Produkts, das die folgenden Schritte umfasst:
- Bereitstellen eines Sprühkopfs (1) einer Vorrichtung zum Sprühen eines fluiden pharmazeutischen Produkts, wobei der Sprühkopf (1) eine einzige Sprühöffnung (2) aufweist,
- Bereitstellen einer Empfangsfläche (10), die eine Vielzahl von Sensoren (20) aufweist,
- Durchleiten eines Druckgasstroms (F) durch die Sprühöffnung (2) des Sprühkopfs (1),
- Leiten des Druckgasstroms (F) auf die Empfangsfläche (10),
- Visualisieren des Auftreffbereichs des Druckgasstroms (F) auf der Empfangsfläche (10) mit Hilfe der Sensoren (20), und
- Analysieren der Visualisierung des Auftreffbereichs, um zu bestimmen, ob der Auftreffbereich vorbestimmten Spezifikationen entspricht oder nicht,
**dadurch gekennzeichnet, dass** die Empfangsfläche (10) zumindest im Bereich des Auftreffbereichs des Druckgasstroms (F) kugelförmig ist,
wobei der Analyseschritt die Bestimmung der Geometrie, insbesondere der Symmetrie, des Auftreffbereichs des Druckgasstroms (F) auf der Empfangsfläche (10) umfasst.

2. Verfahren nach Anspruch 1, wobei der Druckgasstrom (F) ein Druckluftstrom ist.

3. Verfahren nach einem der vorhergehenden Ansprüche, wobei die vorbestimmten Spezifikationen eine vorbestimmte planare Ausdehnung des Auftreffbereichs des Druckgasstroms (F) auf der Empfangsfläche (10) derart umfassen, dass die Sprühköpfe (1), für die die planare Ausdehnung der vorbestimmten planaren Ausdehnung ähnelt, als konform eingestuft werden und die Sprühköpfe (1), für die die planare Ausdehnung von der vorbestimmten planaren Ausdehnung abweicht, als nicht konform eingestuft werden.

4. Verfahren nach einem der vorhergehenden Ansprüche, wobei die Empfangsfläche (10) von der Innenfläche eines Kugelabschnitts, insbesondere einer Halbkugel, gebildet wird.

5. Verfahren nach einem der vorhergehenden Ansprüche, wobei die Empfangsfläche (10) eine Vielzahl von Öffnungen (11) aufweist, die jeweils mit einem entsprechenden Sensor (20) verbunden sind.

6. Verfahren nach einem der vorhergehenden Ansprüche, wobei die Sensoren (20) ein Wabenmuster mit mindestens zwei konzentrischen Sensorbereichen bilden.

7. Vorrichtung zur Analyse einer Vorrichtung zum Sprühen eines fluiden pharmazeutischen Produkts, umfassend:
- einen Sprühkopf (1) einer Vorrichtung zum Sprühen eines fluiden pharmazeutischen Produkts, wobei der Sprühkopf (1) eine einzige Sprühöffnung (2) aufweist,
- eine Empfangsfläche (10), die eine Vielzahl von Sensoren (20) aufweist,
- Mittel zum Erzeugen (50) eines Druckgasstroms (F) zum Durchleiten eines Druckgasstroms (F) durch die Sprühöffnung (2) des Sprühkopfs (1) auf die Empfangsfläche (10),
- Visualisierungsmittel zum Visualisieren des Auftreffbereichs des Druckgasstroms (F) auf der Empfangsfläche (10) mit Hilfe der Sensoren (20), und
- Analysemittel (40) zum Analysieren der Visualisierung des Auftreffbereichs, um zu bestimmen, ob der Auftreffbereich vorbestimmten Spezifikationen entspricht oder nicht, **dadurch gekennzeichnet, dass** die Empfangsfläche (10) zumindest im Bereich des Auftreffbereichs des Druckgasstroms (F) kugelförmig ist,
wobei die Sensoren (20) ein Wabenmuster mit mindestens zwei konzentrischen Sensorbereichen bilden.

8. Vorrichtung nach Anspruch 7, wobei der Druckgasstrom (F) ein Druckluftstrom ist.

9. Vorrichtung nach Anspruch 7 oder 8, wobei die Empfangsfläche (10) von der Innenfläche eines Kugelabschnitts, insbesondere einer Halbkugel, gebildet wird.

10. Vorrichtung nach einem der Ansprüche 7 bis 9, wobei die Empfangsfläche (10) eine Vielzahl von Öffnungen (11) aufweist, die jeweils mit einem entsprechenden Sensor (20) verbunden sind.

11. Vorrichtung nach einem der Ansprüche 7 bis 10, wobei vor jedem Sensor (20) ein Filter (30) angeordnet ist.

12. Vorrichtung nach einem der Ansprüche 7 bis 11, wobei das Netz aus Sensoren (20) einen zentralen Bereich mit einem einzigen zentralen Sensor und einen ersten konzentrischen Bereich mit sechs Sensoren umfasst, der radial außerhalb des zentralen Bereichs angeordnet ist.

13. Vorrichtung nach Anspruch 12, wobei das Netz aus Sensoren (20) einen zweiten konzentrischen Bereich mit zwölf Sensoren umfasst, der radial außerhalb des ersten konzentrischen Bereichs angeordnet ist.

14. Vorrichtung nach Anspruch 13, wobei das Netz aus Sensoren (20) einen dritten konzentrischen Bereich mit achtzehn Sensoren umfasst, der radial außerhalb des zweiten konzentrischen Bereichs angeordnet ist.

15. Vorrichtung nach einem der Ansprüche 7 bis 14, wobei die Sensoren (20) nahe beieinander liegen und so ein regelmäßiges und dichtes Netz aus Sensoren (20) auf der Empfangsfläche (10) bilden.

16. Vorrichtung nach einem der Ansprüche 7 bis 15, wobei die Mittel (50) zum Erzeugen des Druckgasstroms (F) geeignet sind, Impulse mit einstellbarer Dauer, insbesondere von 50 bis 300 ms, zu erzeugen.

17. Vorrichtung nach einem der Ansprüche 7 bis 16, wobei die Sensoren (20) Massendurchflusssensoren sind.

## Claims

1. A method for analysing a fluid product spray device, the method comprising the following steps:
- providing a spray head (1) for a device for spraying a pharmaceutical fluid product, said spray head (1) comprising a single spray orifice (2),
- providing a receiving surface (10) comprising a plurality of sensors (20),
- passing a flow of compressed gas (F) through said spray orifice (2) of said spray head (1),
- sending said flow of compressed gas (F) onto said receiving surface (10),
- visualising the impact zone for said flow of compressed gas (F) on said receiving surface (10) by means of said sensors (20), and
- analysing said visualisation of said impact zone in order to determine whether or not said impact zone complies with predetermined specifications,
**characterised in that** said receiving surface (10) is spherical in shape at least in the impact zone of said flow of compressed gas (F), said step for analysis comprising determining the geometry, in particular the symmetry, of the impact zone for said flow of compressed gas (F) on said receiving surface (10).

2. The method according to claim 1, wherein said flow of compressed gas (F) is a flow of compressed air.

3. The method according to any one of the preceding claims, wherein said predetermined specifications comprise a predetermined planar extent of the impact zone for said flow of compressed gas (F) onto said receiving surface (10), in a manner such that the spray heads (1) for which said planar extent is similar to said predetermined planar extent are classified as compliant, and the spray heads (1) for which said planar extent is different from said predetermined planar extent are classified as non-compliant.

4. The method according to any one of the preceding claims, wherein said receiving surface (10) is formed by the inner surface of a part of a sphere, in particular a half-sphere.

5. The method according to any one of the preceding claims, wherein said receiving surface (10) comprises a plurality of openings (11), each being connected to a respective sensor (20).

6. The method according to any one of the preceding claims, wherein said sensors (20) form a honeycomb array comprising at least two concentric sensor zones.

7. A device for analysing a pharmaceutical fluid product spray device, comprising:
- a spray head (1) for a device for spraying a pharmaceutical fluid product, said spray head (1) comprising a single spray orifice (2),
- a receiving surface (10) comprising a plurality of sensors (20),
- means (50) for generating a flow of compressed gas (F) in order to pass a flow of compressed gas (F) through said spray orifice (2) of said spray head (1) onto said receiving surface (10),
- visualisation means in order to visualise through said sensors (20) the impact zone for said flow of compressed gas (F) onto said receiving surface (10), and
- means (40) for analysis for the analysis of said visualisation of said impact zone in order to determine whether or not said impact zone complies with predetermined specifications,
**characterised in that** said receiving surface (10) is spherical in shape at least in the impact zone said flow of compressed gas (F), said sensors (20) forming a honeycomb array comprising at least two concentric sensor zones.

8. The device according to claim 7, wherein said flow of compressed gas (F) is a flow of compressed air.

9. The device according to claim 7 or claim 8, wherein said receiving surface (10) is formed by the inner surface of a part of a sphere, in particular a half-sphere.

10. The device according to any one of the 7 to 9 claims, wherein said receiving surface (10) comprises a plurality of openings (11), each being connected to a respective sensor (20).

11. The device according to any one of claims 7 to 10, wherein a filter (30) is arranged in front of each sensor (20).

12. The device according any one of claim 7 to 11, wherein said sensor array (20) comprises a central area with a single central sensor, and a first concentric area, arranged radially outside of said central area, with six sensors.

13. The device according to claim 12, wherein said sensor array (20) comprises a second concentric zone, arranged radially outside of said first concentric zone, with twelve sensors.

14. The device according to claim 13, wherein said sensor array (20) comprises a third concentric zone, arranged radially outside of said second concentric zone, with eighteen sensors.

15. The device according to any one claim 7 to 14, wherein said sensors (20) are close to one another, thereby forming a regular and dense array of sensors (20) on said receiving surface (10).

16. The device according to any one of claims 7 to 15, wherein said means (50) for generating a flow of compressed gas (F) are adapted to generate pulses of adjustable duration, in particular from 50 to 300 ms.

17. The device according to any one of claims 7 to 16, wherein said sensors (20) are mass flow sensors.
